# EUROPEAN PATENT SPECIFICATION

(11) **EP 0 580 790 B1**
(45) Date of publication and mention of the grant of the patent: **26.06.2002**
(21) Application number: 92912816.3
(22) Date of filing: 15.04.1992
(51) Int. Cl.: A61K 31/47, A61K 31/475, A61P 35/00

(54) **METHOD FOR POTENTIATING PRIMARY DRUGS IN TREATING MULTIDRUG RESISTANT CELLS**
VERFAHREN ZUR WIRKUNGSVERSTÄRKUNG VON MITTELN DER ERSTEN WAHL BEI DER BEHANDLUNG MEHRFACHRESISTENTER ZELLEN
PROCEDE DE POTENTIALISATION D'UN MEDICAMENT PRINCIPAL DESTINE AU TRAITEMENT DE CELLULES RESISTANTES A LA POLYCHIMIOTHERAPIE

(30) Priority: 19.04.1991 US 689003
(43) Date of publication of application: 02.02.1994
(73) Proprietor: CBA INTERNATIONAL, INC, Lexington, KY 40594 (US)
(72) Inventor: VAN DYKE, Knox, Morgantown, WV 26505 (US)
(74) Representative: Lee, Nicholas John
(86) International application number: US9203085
(87) International publication number: WO9218131

(56) References cited:
- THE JOURNAL OF BIOLOGICAL CHEMISTRY vol. 265, no. 13 , 5 May 1990 pages 7679 - 7686 CHAMBERS, T.C. ET AL 'PROTEIN KINASE C PHOSPHORYLATES P-GLYCOPROTEIN IN MULTIDRUG RESISTANT HUMAN KB CARCINOMA CELLS'
- BIOPHYSICAL AND BIOCHEMICAL RESEARCH COMMUNICATIONS vol. 159, no. 1 , 28 February 1989 pages 242 - 248 YE, Z. ET AL 'SELECTIVE ANTIMALARIAL ACTIVITY OF TETRANDRINE AGAINST CHLOROQUINE RESISTANT PLASMODIUM FALCIPARUM'
- BIOCHEMICAL AND BIOPHYSICAL RESEARCH COMMUNICATIONS vol. 165, no. 2 , 15 December 1989 pages 758 - 765 YE, Z. ET AL 'THE POTENTIATING ACTION OF TETRANDRINE IN COMBINATION WITH CHLOROQUINE OR QINGHAOSU AGAINST CHLOROQUINE-SENSITIVE AND RESISTANT FALCIPARUM MALARIA'
- DATABASE BIOSIS BIOSCIENCES INFORMATION SERVICE, PHILADELPHIA, PA, US AN:79:166230 1978, TOLKACHEV, O.N. ET AL 'THE DEPENDENCE OF THE TUBERCULOSTATIC ACTIVITY OF BIS BENZYL ISO QUINOLINE ALKALOIDS ON THEIR STRUCTURE' & FARMATSIYA vol. 27, no. 2 , 1978 pages 23 - 26
- DATABASE CHEMABS CHEMICAL ABSTRACTS SERVICE, COLUMBUS, OHIO, US AN:CA78(17):106079 1973, VICHKANOVA,S.A. ET AL 'CHEMOTHERAPEUTIC PROPERTIES OF THE ALKALOID TETRANDRINE IN EXPERIMENTAL TUBERCULOSIS' & FARMAKOL.TOKSIKOL. vol. 36, no. 1 , 1973 pages 74 - 78
- DATABASE CHEMABS CHEMICAL ABSTRACTS SERVICE, COLUMBUS, OHIO, US AN:78(11)66905 1972, VICHKANOVA,S.A. ET AL 'TUBERCULOSTATIC ACTIVITY OF PREPARATIONS FROM PLANTS' & FITONTSIDY, MATER. SOVESHCH., 6TH MEETING 1969 pages 90 - 94
- DATABASE CHEMABS CHEMICAL ABSTRACTS SERVICE, COLUMBUS, OHIO, US AN109(19)163208 1988, JIA, JUFANG ET AL 'EFFECTS OF METHOXYADIANTIFOLINE ON CORONARY CIRCULATION AND CONTRACTILITY OF CORONARY STRIPS' & CHIN. J. PHARMACOL. TOXICOL. vol. 2, no. 3 , 1988 pages 193 - 196
- FASEB J vol. 4, no. 7 , 1990 page A2116 MA, J.Y.C. ET AL 'INTERACTION OF BISBENZYLISOQUINOLINE ALKALOIDS WITH ALVEOLAR MACROPHAGES'
- TOXICOLOGY AND APPLIED PHARMACOLOGY vol. 108, no. 2 , 1991 pages 242 - 252 CASTRANOVA, V. ET AL 'EFFECTS OF BISBENZYLISOQUINOLINE ALKALOIDS ON ALVEOLAR MACROPHAGES: CORRELATION BETWEEN BINDING AFFINITY, INHIBITORY POTENCY, AND ANTIFIBROTIC POTENTIAL'
- The Merck Index, 10 Ed., 1985, Nos. 9056 2136, pp. 1321,304.
- CHEMICAL ABSTRACTS, Volume 75, No. 11, issued 1971, September 10 (Columbus, Ohio, USA), Abstract No. 59822w, MITSCHER et al., "Antibiotics from higher Plants. Thalictrum rugosum. New Bisbenzylisoquinolone Alkaloids active against Mycobacterium smeomatis", (Div. Nat. Prod. Chem., Ohio State Univ., Columbus, Ohio), J. Chem Soc. D., 1971, Vol. 11, pp. 589-90.
- CHEMICAL ABSTRACTS, Volume 110, No. 7, issued 1989, March 18 (Columbus, Ohio, USA), FOURNET et al., "Antiparasitic Activity of Bisbenzylisoquinoline Alkaloids. I. In Vitro Activity on the Promastigotes of Three Strains of Leishmania", Abstract No. 1320574 (Orstom, La Paz, Bolivia), J. Ethnopharmacol. 1988, Vol. 24 (2-3), pp. 327-35.
- Chem. Pharm. Bull., Vol. 24(10), 1976, KURODA et al., "Antitumor Effect of Bisbenzylisoquinoline Alkaloids", pp. 2413-2420.
- Gen. Pharmac., Vol. 21(3), 1990, KAWASHIMA et al., "Structure and Hypotensive Activity Relationships in Stroke-Prone Spontaneously Hypertensive Rats", pp. 343-347.
- KATO et al., Uncl, Vol. 79(3), 1987, "Potentiation of Antitumor Activity of Vincristine by the Biscoclaurine Alkaloid Cepharanthine", pp. 527-532.
- Cancer Research, Volume 47, issued 1987, SHIRAISHI et al., "Effect of Bisbenzyl-isoquinoline (Biscoclaurine) Alkaloids on Multidrug Resistance in K B Human Cancer Cells", see pages 2413-2415.

## Description

The present invention relates to use of compounds in the preparation of an agent for the i*n vivo* treatment of cancer cells having multi-drug resistance.

Multidrug resistance is a phenomenon which has been observed in cancer and in a number of parasitic diseases such as malaria, tuberculosis, Entamoeba histolytica (amoebic dysentery), Trypanosoma (African sleeping sickness), Leishmania and AIDS pneumonia.

A number of diverse drugs have been found effective against such diseases. However in many cases, the initial success of physicians in treating the disease is followed by total failure. Drugs which worked initially become totally ineffective after a period of time. An initial period of remission is often followed by a period of frustration during which nothing seems to be effective against the disease. Death becomes inevitable.

Such multidrug resistance in cancer cells has been associated with an increase in the drug resistant cell of the presence of 150,000 to 170,000 molecular weight glycoproteins. Such P150-170 Kd glycoproteins act as a drug exit pump, to pump disease fighting drugs out of the infected or infecting cells which the drugs are supposed to kill. This glycoprotein pump phenomenon in cancer cells has been reported in a March 1989 Scientific American article by Kartner and Ling. (No concession is made that this publication is prior art as to subject matter contained in the parent applications.) The presence of a very similar glycoprotein pump in drug resistant malaria has also been discovered by the inventor.

It has been reported by Rothenberg and Ling that multidrug resistance in cancer can be reversed by using hydrophobic molecules with two planar aromatic rings and a tertiary basic nitrogen atom with a positive charge at physiologic pH. Journal of the National Cancer Institute, Vol. 81, No. 12, June 21, 1989, on page 907. (No concession is made that this publication is prior art as to subject matter contained in the parent applications.) A representative compound of this class, and indeed apparently a member of this class which has actually been the subject of much experimental work is the drug verapamil, whose structural formula is shown below:

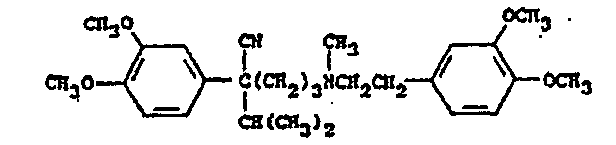

Verapamil is a calcium channel blocker. Other researchers have claimed that calcium channel blockers are effective against malaria. However while such results may be substantiatable in vitro, they have little practical value as clinical treatments in vivo. While calcium channel blockers are therapeutic in the treatment of hypertension at moderate levels, they are toxic at levels high enough to effect MDR reversal.

Another technique for MDR reversal in cancer which is of laboratory interest but which has no practical applicability involves inducing point mutations of the energy related ATP binding sites in the glycoprotein. Such point mutations result in an almost complete loss of MDR activity, according to Rothenberg and Ling, supra. While such in vitro work is important, it lacks in vivo clinical applicability.

Shiraishi *et al* disclose *in vitro* work on the use of cepharanthine to treat multidrug resistance in cancer. Isotetrandrine, tetrandrine, fangchinoline and berbamine are said to show similar effects in cancer. Anti-tumor effects of tetrandrine have also been mentioned. Chambers, T *et al J Biol Chem* **265**:7679 (1990) disclose that tetrandrine has multidrug resistance reversal properties when administered *in vitro* to the KB-V1 cell line.

Heretofore, the phenomenon of multidrug resistance in such disease cells has been attributed to the presence of naturally occurring P150-170 Kd glycoproteins in the disease cells. It is believed that when the disease colony is exposed to treatment, the cells with the glycoprotein, or with a higher percentage thereof, survive the initial treatment while cells without glycoprotein, or with a lesser concentration thereof, do not. The surviving remnant then reproduces and eventually creates a colony which is substantially if not totally resistant to treatment with any drug.

A recent publication in the Proceedings of the National Academy of Science, reveals that multidrug resistance can also be caused by viral infection. "A retrovirus carrying an MDR1 cDNA confers multidrug resistance and polarized expression of P-glycoprotein in MDCK cells," Proc. Natl. Acad. Sci. USA, Ira Pastan et al., Vol. 85, pages 4486-4490, June 1988, Medical Sciences. The authors inserted a full-length cDNA for the human multidrug resistance gene (MDR1) into a retroviral vector. They were able to infect cells with this virus so that the cell expressed P-glycoprotein and rendered the cell multidrug resistant.

This experimental work raises the specter of multidrug resistant infection through accidental release of such manufactured retrovirus. Perhaps more significantly, it suggests the possible natural occurrence of retrovirus carrying such cDNA.

The implications of these possibilities are that diseases normally treatable with drugs initially, including cancers, may become untreatable ab initio due to infection with such multidrug resistance carrying virus. Such virus could through natural or artificial means infect body cells which may later become cancerous, or could infect the cells of parasitic diseases such as malaria, tuberculosis, AIDS pneumonia, African sleeping sickness and other such diseases. The presence of sizable colonies of such multidrug resistant disease cells would render the diseases and cancers particularly aggressive and virulent and very possibly baffling to an unsuspecting physician.

Researchers throughout the world continue to press for techniques for reversing multidrug resistance. A successful clinical technique for reversing multidrug resistance will be one of the most important breakthroughs in the fight against cancer, malaria, tuberculosis and other diseases exhibiting the multidrug resistance phenomenon.

### SUMMARY OF THE INVENTION

In the present invention it has been surprisingly found that tetrandrine and certain of its derivatives act not only to reverse multidrug resistance but also to potentiate the effectiveness of a primary drug against a drug resistant cell. These compounds appear to reverse or inhibit the glycoprotein pump of a multidrug resistant cell so that such a resistant cell actually accepts a greater concentration of drug than a so-called drug resistant cell.

According to the present invention, there is provided the use of a compound of general formula I 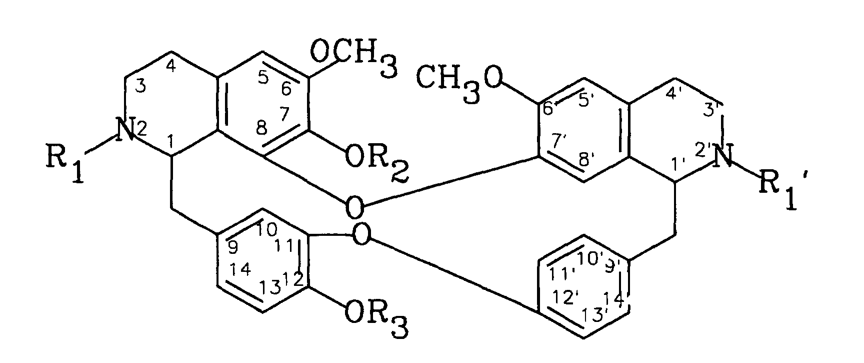 wherein R₁ and R₁' are the same or different and are H, CH₃ or CO₂CH₃;
R₂ is CH₃;
R₃ is CH₃ or hydrogen;
the isomeric configuration at the C-1' chiral carbon location is "S"; and
at least one of R₂ and R₃ comprises CH₃;
in the preparation of an agent for the *in vivo* treatment of cancer cells having multidrug resistance.

The tetrandrine family of compounds as a whole includes tetrandrine, isotetrandrine, hernandezine, berbamine, pycnamine, phaeanthine, obamegine and fangchinoline, which list is not intended to be exhaustive. In all of these examples, R₁ and R₁' constitute the methyl group. Variation within the group occurs in that R₂ and R₃ may constitute either a methyl group or hydrogen, and the isomeric configuration of the compounds at the C-1 chiral carbon position is either R (rectus) or S (sinister). The rules for R and S configuration can be found in Morrison and Boyd, "organic chemistry," 4th Edition, copyright 1983 by Allyn and Bacon, at pages 138-141. In addition, hernandezine includes a methoxy group at the C-5 position, a substitution which does not appear to be significant in the operability of the compound in the present invention. The specific manner in which these exemplary family members vary is set forth in Table VII below, wherein these family members are compared to two nonfamily members for activity against drug sensitive and drug resistant strains of P. falciparum malaria.

Not all members of the tetrandrine family of compounds operate to enhance or potentiate the activity of a primary drug against a multidrug resistant cell. only those members of the family having the specific configuration outlined above are operable in this manner. Of the eight representative members of the family above, only tetrandrine, isotetrandrine, hernandezine and berbamine act to potentiate the primary drug against multidrug resistant cells.

These compounds actually make the drug resistant cell more sensitive to the inhibitory action of a drug than is the so-called drug sensitive cell. At present, the only logical explanation for this result is that the compounds actually reverse the glycoprotein pumps which are found in greater abundance on drug resistant cells. Another explanation is that multidrug resistant cells are actually more sensitive than drug sensitive cells to the primary toxic drug but this cannot be seen until the exit pump is inhibited. Thus the glycoprotein pump mechanism which originally made the cell multidrug resistant to drug inhibition actually works against the cell to make the cell more sensitive to drug inhibition.

A specific in vivo dosage for each of the various compounds used in the present invention has not been established. However, such dosage can be established through routine clinical experimentation by referencing the concentrations at which the various compounds have exhibited 50% inhibition as set forth in Tables III through VII herein. These conoentrations have been from about .1 to about 3 micro molar. Such concentrations can be achieved in vivo by administering dosages of from about 100 to about 300 mg/day. It is known that at these concentrations, tetrandrine is substantially nontoxic. The preferred method for administering the drug is orally, though other methods such as injection may be used.

In the treatment of various canoers whose cells are multi-drug resistant, a member of the tetrandrine family as described above, or mixtures thereof, is administered in conjunction with primary drugs known to have effectiveness against particular cancers. The following are anti-cancer drugs which are pumped by the multidrug resistance mechanism: doxorubicin, vinblastine, vincristine, adriamycin, mythramycin, actinomycin D, colchicine, daunomycin, mitoxantrone, VP-16 (podophyllotoxin).

The invention will be described further in the following non-limiting examples, which refer to the accompanying drawings, in which:
Figure 1 is a graph comparing different concentrations of doxorubicin when used against drug resistant DX20, alone and in combination with different concentrations of tetrandrine, and when used against drug sensitive parent MES-SA cells;
Figure 2 is a graph comparing different concentrations of vincristine when used against drug resistant DX20, alone and in combination with different concentrations of tetrandrine, and when used against drug sensitive parent MES-SA cells;
Figure 3 is a graph comparing different concentrations of vinblastine when used against drug resistant DX20, alone and in combination with different concentrations of tetrandrine, and when used against drug sensitive parent MES-SA cells;
Figure 4 is a graph comparing different concentrations of doxorubicin when used against drug resistant A2780AD10, alone and in combination with different concentrations of tetrandrine, and when used against drug sensitive parent A2780 cells;
Figure 5 is a graph comparing different concentrations of doxorubicin when used against drug resistant DX20, alone and in combination with different concentrations of tetrandrine, and when used against drug sensitive parent MES-SA cells;
Figure 6 is a graph demonstrating the effect doxorubicin and tetrandrine used as a single agent have on tumor size;
Figure 7 is an isobologram showing the effectiveness of tetrandrine and chloroquine at 50% inhibition concentrations against sensitive and resistant malarial strains; and
Figure 8 is an isobologram showing the effectiveness of tetrandrine and qinghaosu at 50% inhibition concentrations against sensitive and resistant malarial strain.

### EXAMPLE 1

The effectiveness of tetrandrine in potentiating anticancer drugs in multidrug resistant cancer cells was determined with respect to three drug combinations: tetrandrine and doxorubicin; tetrandrine and vincristine; and tetrandrine and vinblastine. These combinations were investigated in the following multiple drug resistant human cancer cell lines: MES-SA/DX20 (the parent and doxorubicin-resistant cell lines from uterine leiomyosarcoma) and A2780/A2780AD10 (the parent and multiple drug resistant cell lines from an individual with ovarian cancer). The MES-SA/DX20 cell line was established according to the development methods of Sikic et al., Resistance to Antineoplastic Drugs, Chap. 3, CRC Press, Ed. David Kessel, pp. 37-47 (1989). In vitro drug sensitivity testing consisted of two assay models, the modified MTT assay (Mossmann, J. Immunolog. Methods, Vol. 65, pp. 55-63, 1983) and the clonogenic assay. In addition to this, the combinations were investigated in vitro in nude mice bearing the DX20 cell line. The MTT assay was chosen because of its practical advantages in large-scale screening. The assay involved cellular conversion of tetrazolium salt to a colored formazan serving as a measurement of cell viability.

### Modified MTT assay

Culture media, 50 microliters, containing 8,000-25,000 cells, depending on the proliferation rate of the cell line, was pipetted into each well of several 96-well microtiter plates. The cells were allowed to attach overnight. 100 microliters of d-Tetrandrine-(S) solution, at a concentration below that established as an independent IC₅₀, was then added to each well of the plates. The cytotoxic drug, also at a concentration below that previously established as an IC₅₀, was then added at decreasing concentrations by 25% to several half plate sections. The cells were allowed to grow for 72 hours. 100 microliters of media was then removed and 25 microliters of 5 mg/ml MTT (sigma, St. Louis, MO) in phosphate buffered saline was added to each well. The plates were kept in an incubator at 37°C and 5% CO₂. After 2-3 hours of incubation, the extraction buffer containing 20% w/v SDS and 50% DMF was added and the plates again incubated overnight. The plates were read at a test wavelength of 570 nm on a multiwell spectrophotometer (Model MR 700, Dynatech Laboratories, Alexandria, VA). Each drug combination was tasted in triplicate. Percent survival was defined as percent of optical density (OD) of the drug-treated wells to that of the control detected by the spectrophotometer.

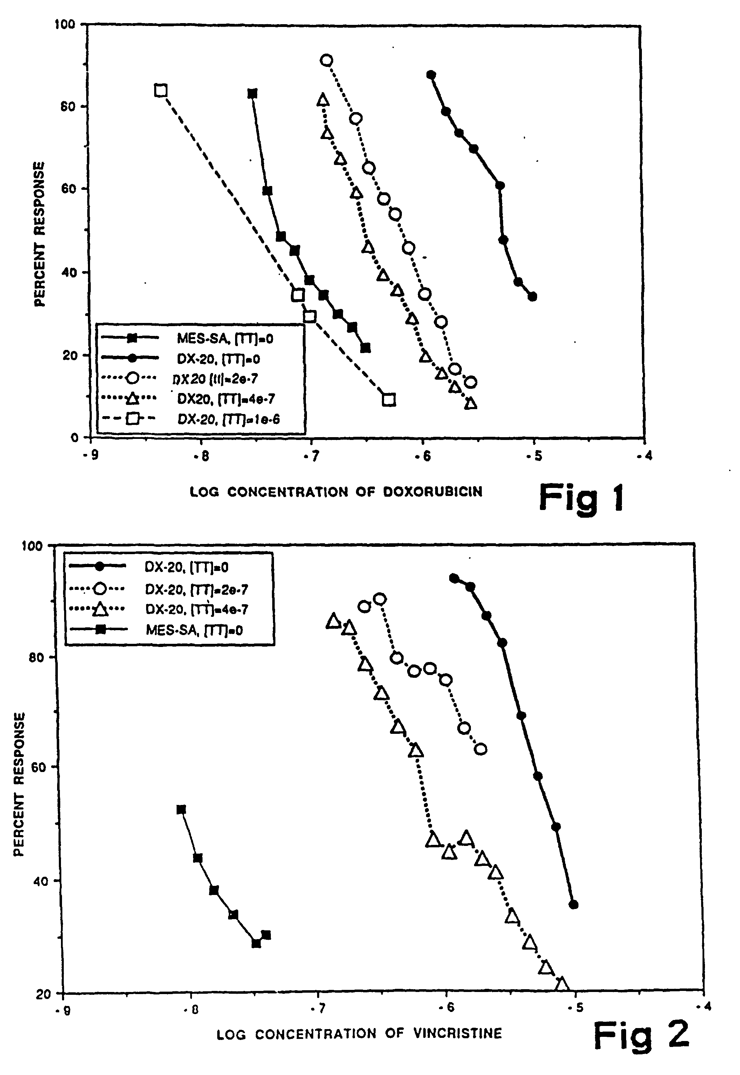

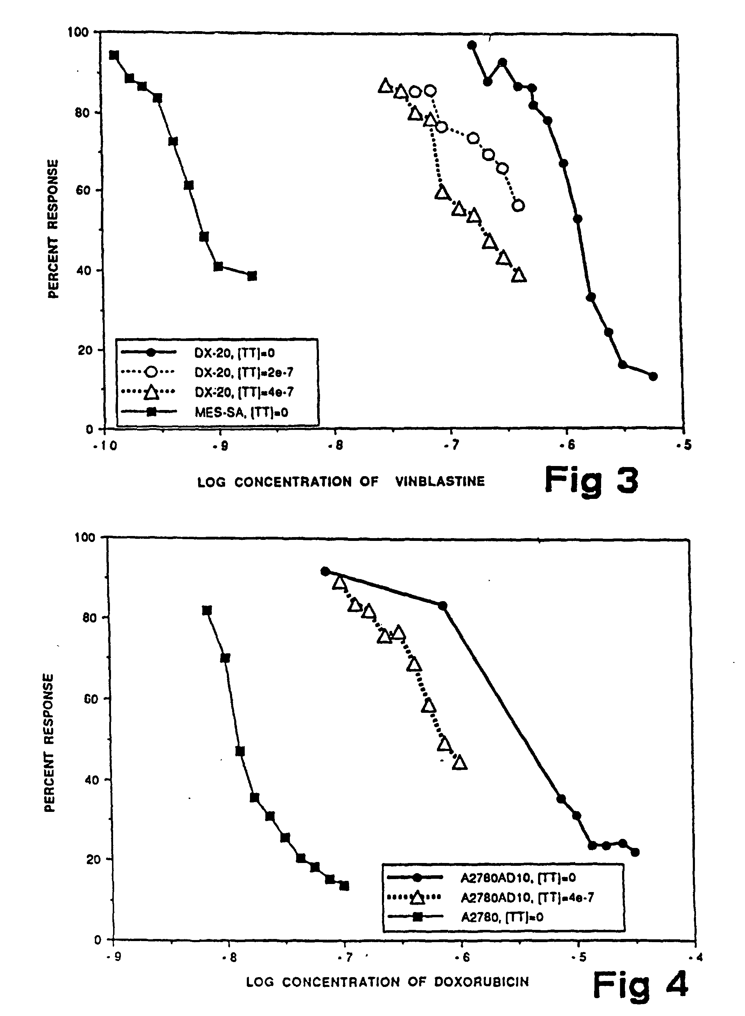

Figures 1, 4 and 5 demonstrate that doxorubicin-resistance can be either partially or completely reversed with the addition of tetrandrine. Figure 1 demonstrates that when tetrandrine at 1.0 x 10⁻⁶ M is combined with doxorubicin at 1.2 x 10⁻⁸ M there is a dramatic shift to the left. This shift demonstrates that the DX20 doxorubicin- resistant cell line becomes more sensitive to doxorubicin than the parent (MES) cell line. The calculated IC₅₀ values also demonstrate that if the tetrandrine dose is increased from 2 x 10⁻⁷ to 1 x 10⁻⁶ the doxorubicin dose can be decreased from 6.6 x 10⁻⁷ to 3.4 x 10⁻⁸. Figures 2 and 3 demonstrate that vincristine-resistance and vinblastine resistance can be either partially or completely reversed with the addition. of tetrandrine as well.

### Clonogenic assay

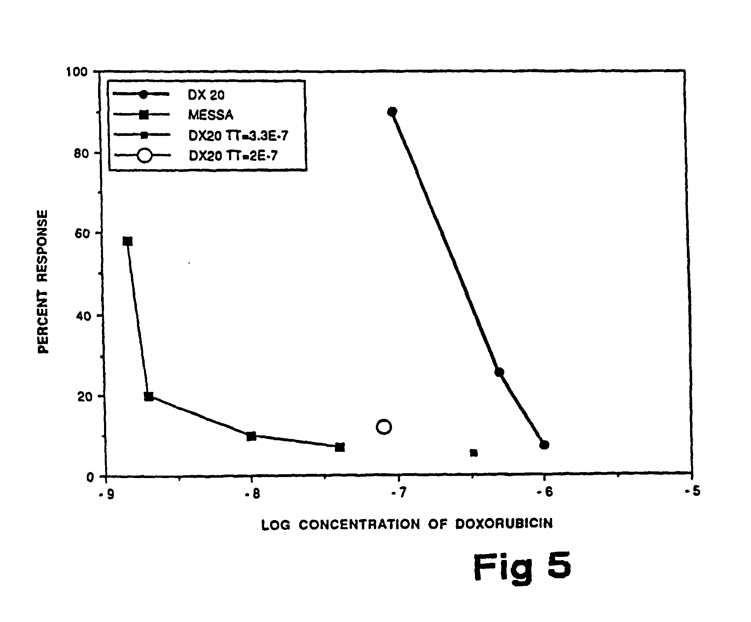

The second assay model used, colony formation assay, consisted of 1000-1500 cells, again depending on the proliferation rate of the cell line being added to each well of 24 well microtiter plates in 500 micoliters of culture media. The cells were allowed to attach overnight. As in the MTT, culture media containing d-Tetrandrine-(S) and also a cytotoxic drug were added at concentrations below those previously established as independent IC₅₀ values. The plates were incubated for a total of 7 days before being fixed in 2.5% glutaraldehyde and stained with crystal violet. Quantitations of colony growth were assessed using an ultrasensitive imaging device (LCVS-5, I.P.S., Pittsburgh, PA). A colony is defined as any cluster of 30 or more cells. Percent inhibition was directly measured against control plates containing no drugs. Figure 5 suggests also that doxorubicin-resistance can be either partially or completely reversed with the addition of tetrandrine.

### Nude mice bearing the DX20 cell line

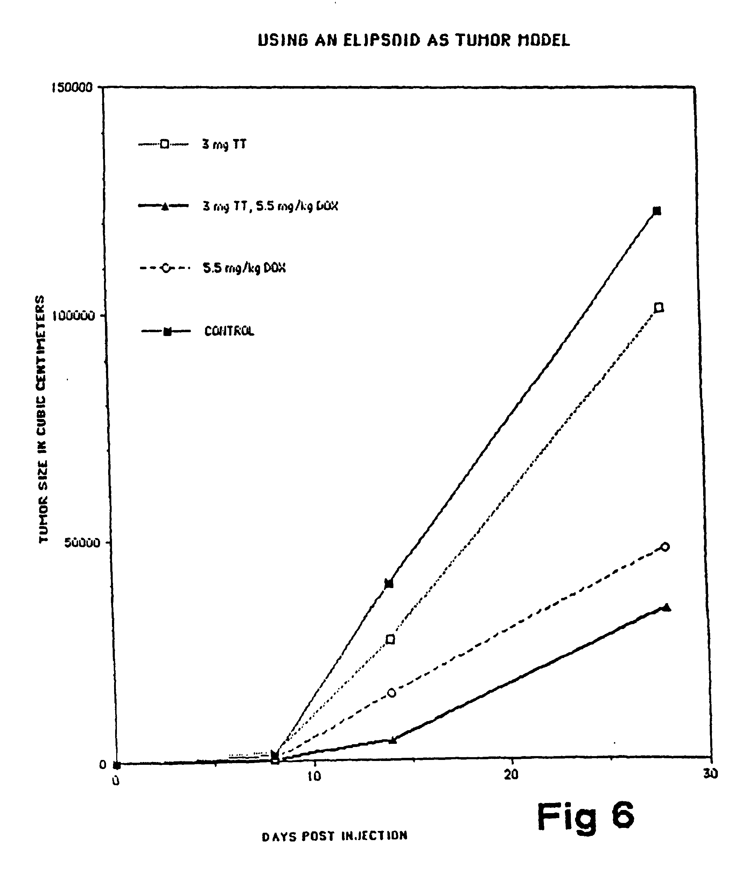

In addition, the effects of d-Tetrandrine-(S) plus doxorubicin in Nu/Nu athymic mice inoculated with DX20 sarcoma cells, 76 x resistant when compared to the parent MES-SA cells were examined. Four cohorts of nude animals bearing the DX20 tumor were treated in groups of 5 (control, doxorubicin, tetrandrine, and doxorubicin + tetrandrine). Initial data was gathered by monitoring tumor growth, comparing visible retardation in experimental groups receiving 2 cycles of d-Tetrandrine-(S) and doxorubicin over those receiving either d-Tetrandrine-(S) or doxorubicin alone in comparable amounts. (See Fig. 6). Tumor size was calculated using the formula (4 Pi/3) X * Y * 2Z. Additional analysis showed that when given in 3 intraperitoneal injections over 120 hours, 150 mg/kg total, 54 micrograms/gram tissue d-Tetrandrine-(S) could be detected at the tumor. An L.D.₅₀ dose of 260 mg/kg was also calculated by administering single escalating injections of the compound. Tables I & II and Figure 6 give the actual measurements.

**TABLE I**

| | Tumor Measurements on Day 7 Prior to Treatment | | |
|---|---|---|---|
| | Length | Width | Depth |
| Control | 3.66 | 3.33 | 1.5 |
| Doxorubicin | 3.50 | 3.87 | 1.25 |
| Tetrandrine | 4.13 | 3.75 | 1.13 |
| Doxorubicin + Tetrandrine | 3.75 | 4.37 | 1.62 |

**TABLE II**

| | Tumor Measurements on Day 24 After Treatment | | |
|---|---|---|---|
| | Length | Width | Depth |
| Control | 30.50 | 25.00 | 21.50 |
| Doxorubicin | 24.50 | 19.10 | 21.50 |
| Tetrandrine | 30.60 | 26.16 | 24.66 |
| Doxorubicin + Tetrandrine | 18.00 | 13.62 | 12.75 |

The study was done with 3-4 week old male balb-C nude mice. The animals were injected with DX20 1 x 10⁷ cells. The animals were treated with tetrandrine on days 6, 8, 10 (1.5 mg, 1 mg, and 0.5 mg). The mice were given doxorubicin 5.5 mg/kg on day 12.

### REFERENCE EXAMPLE 1

The effectiveness of tetrandrine in potentiating antimalarial drugs in multidrug resistant parasitic malarial cells was determined by comparing the antimalarial action of tetrandrine and chloroquine alone and in combination against a P. falciparum malarial strain which is sensitive to chloroquine and another which is resistant to chloroquine. A similar study was conducted using tetrandrine and qinghaosu. Chloroquine and qinghaosu are commonly used antimalarial drugs.

The dose (IC₅₀) of each drug or each drug combination required to effect a 50% inhibition in the malarial activity of each strain was determined by establishing a dose response curve for each.

FCMSU1/Sudan strain and cloned Indochina (W-2) strain of P. falciparum were used. The former is sensitive to chloroquine and the latter is resistant to chloroquine. The two strains of the parasite were cultured according to the candle jar method of Trager and Jensen, Science, Vol. 193, pages 673-675 (1976). In a given experiment, four-day-old Petri dish cultures (approximately 10% parasitemia) were diluted with medium containing an amount of noninfected type A human erythrocytes to obtain a culture with a final hematocrit of 1.5% and parasitemia of 0.5-1.0%. The resulting culture was ready for addition to microtitration plates with ninety-six flat-bottom wells.

The testing procedure used was similar to that described by Desjardins et al. in "Antimicrobial Agents and Chemotherapy," Vol. 16, pages 710-718 (1979). Briefly, the final volume added to each of the ninety-six well microtitration plates was 250 microliters and consisted of 25 microliters of complete medium with or without the primary drug (chloroquine or qinghaosu), 175 mioroliters of either the parasitized culture or a nonparasitized human erythrocyte control, and 25 microliters of complete medium with or without tetrandrine. 25 microliters radioactive (0.5 microCi) [2,8-³H] adenosine. The microtitration plates were incubated in a candle jar for an additional 18 hours, at 37° C.

As the malaria parasite grows ³H-adenosine is metabolized and incorporates into polymeric RNA and DNA. The labeled polymers are trapped on glass fiber filters and unincorporated material is washed away. In the absence of drug there is 100% incorporation of the labeled material. When drugs interfere (directly or indirectly), an inhibitory dose of 50% (IC₅₀) can be calculated. The experiments were repeated three times except where noted. Statistical analysis was done using Student's T-test for significance. Van Dyke et al. "Exp. Parasitol," Vol. 64, pages 418-423 (1987).

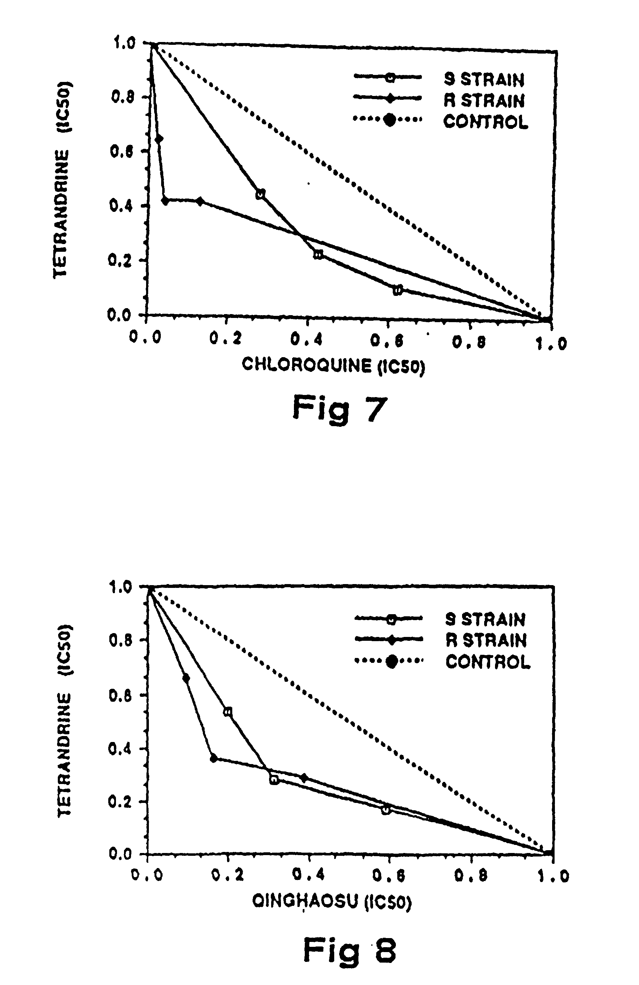

When tetrandrine is added to chloroquine, it supplements and potentiates the antimalarial activity. When tetrandrine is added to qinghaosu or chloroquine, it provides long-acting and synergistic activity to qinghaosu or chloroquine. This can be seen in Tables III-VI and in figures 7 & 8. Remarkably, when 3.0 micromolar tetrandrine is added to 0.1 micromolar chloroquine, the IC₅₀ of chloroquine can be lowered 43-Fold.

**TABLE III**

| IC₅₀ (nM) OF TT AND CO FOR EACH DRUG ALONE AND IN COMBINATION* | | | | | |
|---|---|---|---|---|---|
| MALARIA*** | SINGLE DRUG | | DRUG COMBINATION** | | |
| | TT | CQ | TT(1.0uM) | TT(2.0uM) | TT(3.0uM) |
| | | | CQ(0.3uM) | CQ(0.2uM) | CQ(0.1uM) |
| S STRAIN | 498.1±93.7 | 26.7±3.8 | 54.9±7.1(TT) | 114.1±23.0(TT) | 223.3±38.6(TT) |
| | | | 16.5±2.1(CQ) | 11.4±2.3(CQ) | 7.4± 1.3(CQ) |
| | | | | | |
| R STRAIN | 197.5±24.7 | 185.8±4.9 | 79.5±13.7(TT) | 79.5±18.1(TT) | 124.6± 9.6(TT) |
| | | | 23.8±4.1(CQ) | 8.0± 1.8(CQ) | 4.2± 0.3(CQ) |

| | | | | | |
|---|---|---|---|---|---|
| * The data in the table above are the mean values ± S.D (nM) from three experiments except where noted. | | | | | |
| ** Ratios of TT/CQ in the drug combinations are 10:3, 10:1 and 30:1 respectively. | | | | | |
| *** S and R strains represent CO.sensitive (FCMSU1/Saden) and resistant (w2) strain of P. Falciparum respectively | | | | | |

When the inhibiting activity of two drugs, e.g., A and B are compared, the middle point of the dose response curve is usually chosen as the basis for comparison. This point is known as the inhibitory dose that occurs at the point of 50% inhibition of the response to be measured (inhibitory concentration at 50% inhibitory response = IC₅₀). An isobologram is developed by comparing the IC₅₀ of one drug against the other, i.e., drug A against drug B. We start by putting the IC₅₀ of drug B at the top of the y axis marked 1.0. The IC₅₀ of drug A is placed at the position 1.0 on the x axis. The combinations of drug A and drug B are mixed and tested that are below IC₅₀ of either drug and the points are located on the graph. If the two drugs are additive, there is a straight line between the Y₁X₀ (drug B) and Y₀X₁ (drug A). If the line or curve bends below the straight line, the drugs are synergistic or potentiating. If the line bends above the straight line, the two drugs are antagonistic.

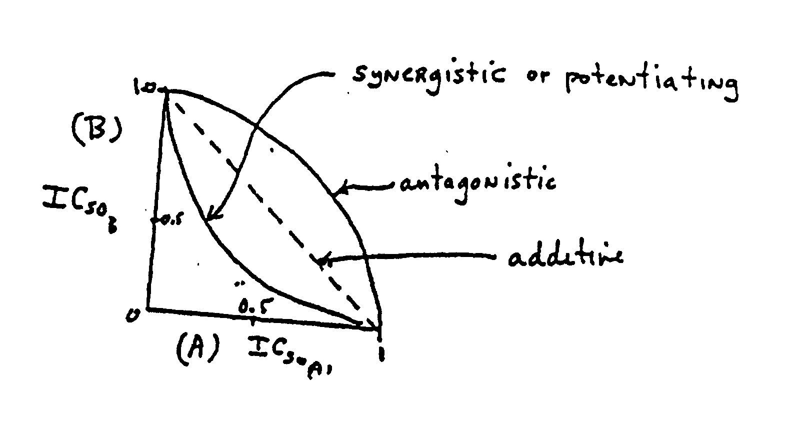

**TABLE IV**

| IC₅₀ (nM) OF TT AND OHS FOR EACH DRUG ALONE AND IN COMBINATION* | | | | | |
|---|---|---|---|---|---|
| MALARIA** | SINGLE DRUG | | DRUG COMBINATION** | | |
| | TT | QHS | TT(1.0uM) | TT(2.0uM) | TT(3.0uM) |
| | | | QHS(0.3uM) | QHS(0.2uM) | QHS(0.1uM) |
| S STRAIN | 410.2±69.0 | 36.7±4.7 | 71.9±8.9(TT) | 113.5±6.3(TT) | 219.5±35.5(TT) |
| | | | 21.6±2.7(QHS) | 11.4±0.5(QHS) | 7.3±1.2(QHS) |
| | | | | | |
| R STRAIN | 205.6±49.8 | 47.8±14.5 | 59.6±13.7(TT) | 71.8±13.8(TT) | 136.9±41.6(TT) |
| | | | 17.9±4.1(QHS) | 7.2±1.4(QHS) | 4.6±1.4(QHS) |

| | | | | | |
|---|---|---|---|---|---|
| * The data in the table above are the mean values ± S.D (nM) from three experiments except where noted. | | | | | |
| ** Ratios of TT/QHS in the drug combinations are 10:3, 10:1 and 30:1 respectively. ***S and R strains represent CO.sensitive (FCMSU1/Sudan) and resistant (W2) strain of P.falcioarum respectively. | | | | | |

**TABLE V**

| EFFECT OF COMBINATION OF TETRANDRINE AND CHLOROQUINE ON P.FALCIPARUM | | | | |
|---|---|---|---|---|
| MALARIA** | TRIAL | SFIC* | | |
| | | 1.0 uM TT | 2.0 uM TT | 3.0 uM TT |
| | | 0.3 uM CQ | 0.2 uM CQ | 0.1 uM CQ |
| S STRAIN | 1 | 0.77 | 0.66 | 0.73 |
| | 2 | 0.64 | 0.77 | 0.70 |
| | 3 | 0.78 | 0.55 | 0.75 |
| | MEAN ± S.D | 0.73 ±0.06 | 0.66 ± 0.09 | 0.73 ± 0.02 |
| | | | | |
| R STRAIN | 1 | 0.60 | 0.45 | 0.74 |
| | 2 | 0.68 | 0.63 | 0.76 |
| | 3 | 0.36 | 0.30 | 0.50 |
| | MEAN ± S.D | 0.55 ±0.14 | 0.46 ± 0.14 | 0.67 ± 0.12 |

| | | | | |
|---|---|---|---|---|
| * SFIC represents sum of fractional inhibitory concentration as described by Berenbaum (11), SFIC is equal to one in cases of additive effects of the drugs, higher then one in cases of antagonism and lower than one in synergistic sc. | | | | |
| ** S and R strain: chloroquine sensitive (FCMSU1/Sudan) and resistant (w2) strain of P.falciparum. | | | | |

**TABLE VI**

| EFFECT OF COMBINATION OF TETRANDRINE AND OINGHAOSU ON P.FALCIPARUM | | | | |
|---|---|---|---|---|
| MALARIA** | TRIAL | SFIC* | | |
| | | 1.0 uM TT | 2.0 uM TT | 3.0 uM TT |
| | | 0.3 uM QHS | 0.2 uM QHS | 0.1 uM QHS |
| S STRAIN | 1 | 0.77 | 0.68 | 0.71 |
| | 2 | 0.74 | 0.49 | 0.72 |
| | 3 | 0.79 | 0.62 | 0.77 |
| | MEAN ± S.D | 0.77 ± 0.02 | 0.60 ± 0.08 | 0.73 ± 0.03 |
| R STRAIN | 1 | 0.63 | 0.46 | 0.71 |
| | 2 | 0.77 | 0.72 | 0.74 |
| | 3 | 0.64 | 0.40 | 0.81 |
| | MEAN ± S.D | 0.58 ±0.06 | 0.52 ± 0.14 | 0.75 ± 0.04 |

| | | | | |
|---|---|---|---|---|
| * SFIC represents sum of fractional inhibitory concentration as described by Berenbaum (11), SFIC is equal to one in cases of additive effects of the drugs, higher than one in cases of antagonism and lower than one in synergistic action. | | | | |
| ** S and R strain: chloroquine sensitive (FCMSU1/Sudan) and resistant (W2) strains of P.falciparum. | | | | |

In an attempt to explain this surprising result, tetrandrine and various of its derivatives and several nontetrandrine derivatives were tested for their individual effectiveness against a chloroquine sensitive and a chloroquine resistant strain of P falciparum malaria. The test procedure was basically the same as outlined above. The nonfamily members were cycleanine, cepharanthine, methoxadiantifoline and thalicarpine, whose structural formulas are illustrated herebelow:

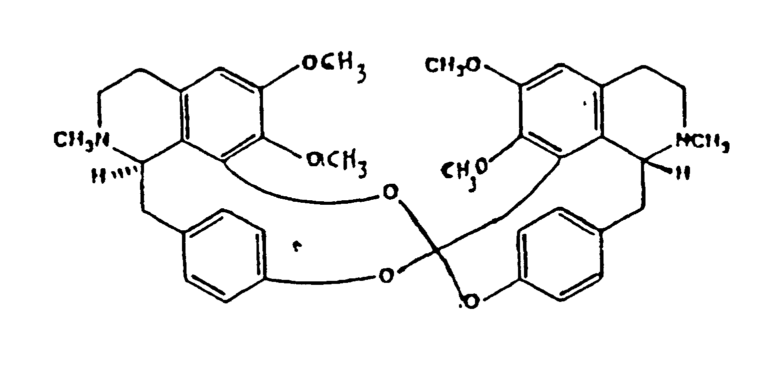

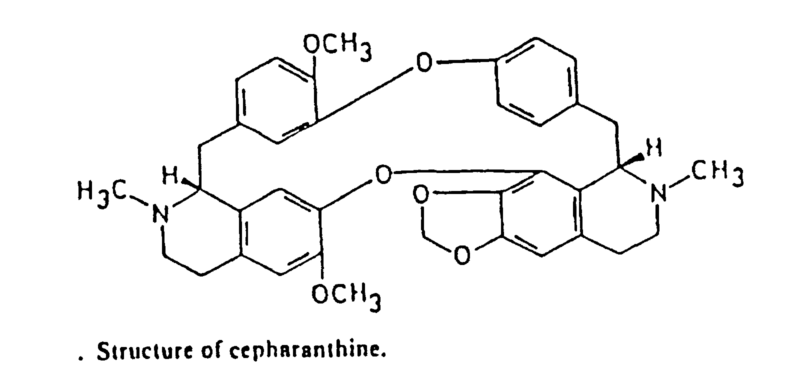

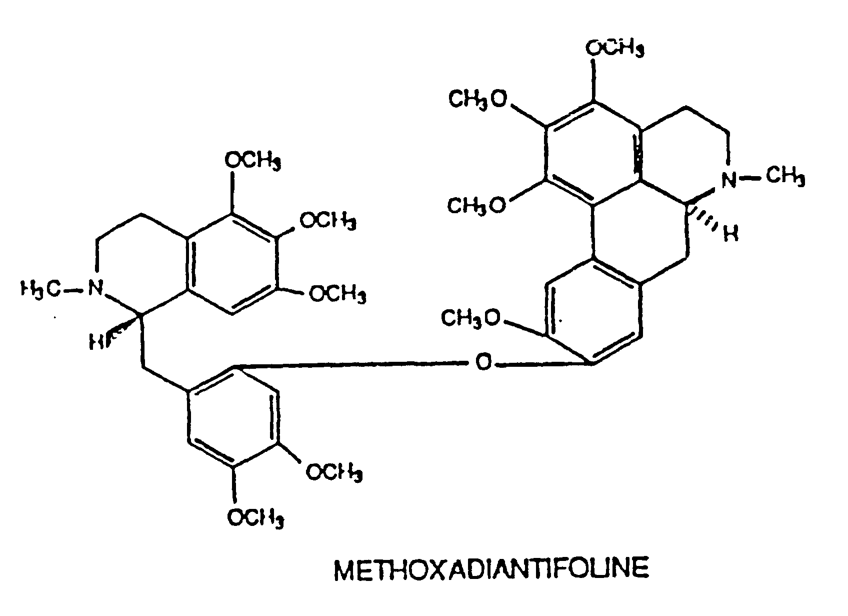

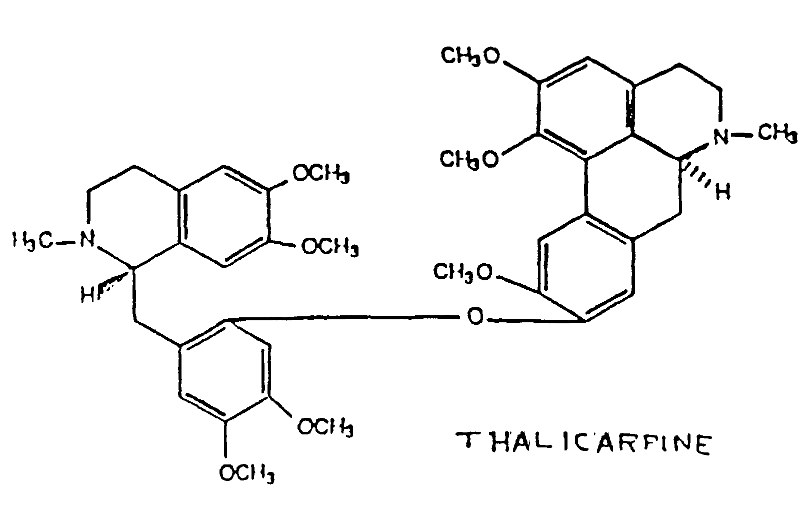

These Comparative Activities are set forth in Table VII Below

| CHEMICAL STRUCTURE · ANTIMALARIAL ACTIVITY OF BISBENLYL ISOQUINOLINE ALKALOIDS AGAINST PLASMODIUM FALCIPARUM IN VITRO | | | | | | | | | | |
|---|---|---|---|---|---|---|---|---|---|---|
| Drug (a) | Configuration | | Substituents | | | | Oxygen Bridge | IC₅₀ (10⁻⁷M) | | Ratio (S/R)* |
| | C-1 | C-1' | C-5 | C-7 | C-12 | C-5' | | S** | R** | |
| TT | S | S | H | OCH3 | OCH3 | | C8-C7' | 2.9 | 1.2 | 2.6 |
| | | | | | | | C11-C12' | | | |
| IT | R | S | H | OCH3 | OCH3 | | C8-7' | 4.8 | 1.4 | 3.5 |
| | | | | | | | C11-C12' | | | |
| HE | S | S | OCH3 | OCH3 | OCH3 | | C8-C7' | 3.7 | 1.3 | 2.8 |
| | | | | | | | C11-C12' | | | |
| BB | R | S | H | OCH3 | OH | | C8-C7' | 4.6 | 1.9 | 2.7 |
| | | | | | | | C11-C12' | | | |
| PY | R | R | H | OCH3 | OH | | C8-C7' | 3.8 | 4.2 | 0.9 |
| | | | | | | | C11-C12' | | | |
| PH | R | R | H | OCH3 | OCH3 | | C8-C7' | 6.0 | 5.0 | 1.2 |
| | | | | | | | C11-C12' | | | |
| OB | R | S | H | OH | OH | | C8-C7' | 6.6 | 4.8 | 1.5 |
| | | | | | | | C11-C12' | | | |
| FA | S | S | H | OH | OCH3 | | C8-C7' | 2.6 | 2.2 | 1.2 |
| | | | | | | | C11-C12' | | | |
| CY | R | R | H | OCH3 | | | C8-C12' | 32 | 42 | 0.8 |
| | | | | | | | C12-C8' | | | |
| CE | S | R | H | OCH2- | | | C8-C7' | 10 | 9.4 | 1.1 |
| | | | | | | | C12-C11' | | | |
| ME | S | S | OCH3 | OCH3 | OCH3 | OCH3 | C10-C12' | 53 | 9.7 | 5.5 |
| TH | S | S | H | OCH3 | OCH3 | H | C10-C12' | 17 | 13 | 1.3 |

| | | | | | | | | | | |
|---|---|---|---|---|---|---|---|---|---|---|
| (a): TT-tetrandrine: TT-isoletrandrine: HE-hernandezine: BB-berbamine: PY-pynamine: PH-phacanthine: OB-obamegine: TA-fangchinoline: CY-cycleanine: CE-cephraanthine: ME-methoxadiantifoline: TH-thalicarpine | | | | | | | | | | |
| * IC₅₀ or a drug against sensitive strain of P.falciparum is devided by IC₅₀ for resistant strain. | | | | | | | | | | |
| ** S and R represent chloroquine-sensitive and resistant strain or P.falciparum. | | | | | | | | | | |

The results of Table VII illustrate that methoxadiantifoline and those members of the tetrandrine family having the "S" isomeric configuration at the C-1' chiral carbon and having at least one of the R₂ substituent comprising CH₃ are actually substantially more effective against the chloroquine resistant malarial strain than against the chloroquine sensitive malarial strain. This extremely surprising result suggests that these compounds actually reverse or inhibit the pumping action of the glycoprotein associated with such multidrug resistant cells. Instead of pumping the toxic drug out of the cell, it actually appears to be pumping a lesser concentration of the toxic drug out of the cell. At present, this is the only reasonable explanation for these surprising results, since the only known significant difference between the multidrug resistant cells and the corresponding drug sensitive cells is the substantially greater percentage of P-glycoprotein associated with the multidrug resistant cell.

The foregoing illustrates that the compounds used in the present invention can provide a treatment for multidrug resistance in cancer cells. The invention is directed to the use of such compounds in the preparation of an agent for the *in vivo* treatment of cancer cells having multidrug resistance. The compounds potentiate the action of primary drugs known to be effective against the multidrug resistance infected disease cells.

## Claims

1. The use of a compound of general formula I 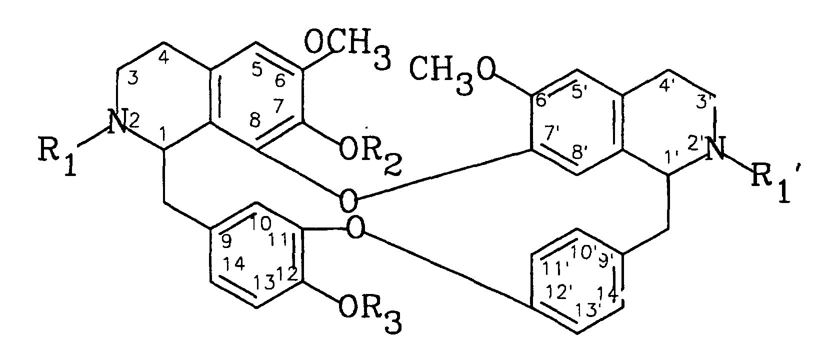 wherein R₁ and R₁' are the same or different and are H, CH₃ or CO₂CH₃;
R₂ is CH₃;
R₃ is CH₃ or hydrogen;
the isomeric configuration at the C-1' chiral carbon location is "S"; and
at least one of R₂ and R₃ comprises CH₃;
in the preparation of an agent for the *in vivo* treatment of cancer cells having multidrug resistance.

2. The use of a compound as claimed in claim 1, wherein the cells are treated with at least one principal drug known to be effective for treating cancer.

3. The use of a compound as claimed in claim 2, wherein the principal drug is doxorubicin, vinblastine, vincristine, adriamycin, mythramycin, actinomycin D, colchicine, daunomycin, mitoxantrone or podopyllotoxin.

4. The use of a compound as claimed in any one of claims 1 to 3 wherein the dosage level is from about 100 to 300 mg per day.

5. The use of a compound as claimed in any one of claims 1-4, wherein the compound is tetrandrine.

## Patentansprüche

1. Die Verwendung einer Verbindung der allgemeinen Formel I 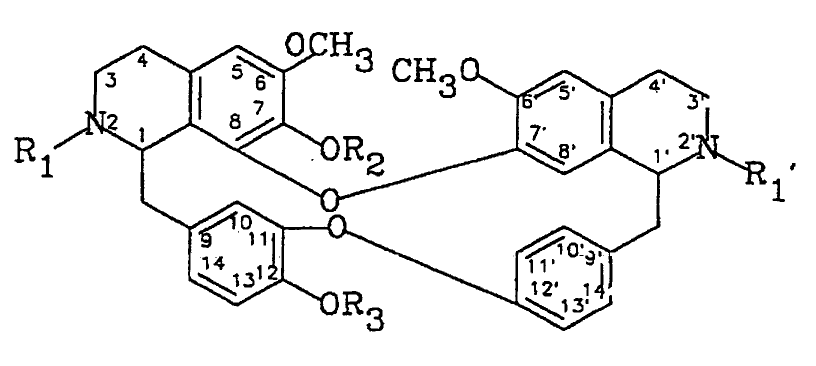 wobei R₁ und R₁' gleich oder verschieden sind und für H, CH₃ oder CO₂CH₃ stehen;
R₂ für CH₃ steht;
R₃ für CH₃ oder Wasserstoff steht;
die isomere Konfiguration an der Stelle des chiralen Kohlenstoffatoms C-1' "S" ist; und
mindestens einer von R₂ und R₃ CH₃ umfasst;
bei der Herstellung eines Mittels für die Behandlung *in vivo* von Krebszellen mit einer Resistenz gegen mehrere Arzneistoffe.

2. Die Verwendung einer Verbindung nach Anspruch 2, wobei die Zellen mit mindestens einem grundsätzlichen Arzneistoff, von dem bekannt ist, dass er bei der Behandlung von Krebs wirksam ist, behandelt werden.

3. Die Verwendung einer Verbindung nach Anspruch 2, wobei der wesentliche Arzneistoff Doxorubicin, Vinblastin, Vincristin, Adriamycin, Mythramycin, Actinomycin D, Colchicin, Daunomycin, Mitoxantron oder Podopyllotoxin ist.

4. Die Verwendung einer Verbindung nach einem der Ansprüche 1-3, wobei die Dosierungshöhe zwischen etwa 100 und 300 mg pro Tag liegt.

5. Die Verwendung einer Verbindung nach einem der Ansprüche 1-4, wobei die Verbindung Tetrandrin ist.

## Revendications

1. Utilisation d'un composé de formule générale I 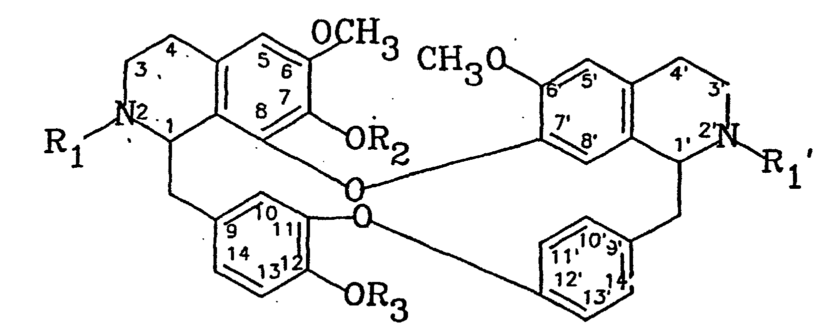 dans laquelle R₁ et R₁' sont identiques ou différents et sont H, CH₃ ou CO₂CH₃ ;
R₂ est CH₃ ;
R₃ est CH₃ ou hydrogène ;
la configuration isomérique au site de carbone chiral C-1' est « S » ; et
au moins un des groupements R₂ et R₃ comprend CH₃ ;
lors de la préparation d'un agent pour le traitement in vivo de cellules cancéreuses résistantes à plusieurs médicaments.

2. Utilisation d'un composé selon la revendication 1, dans laquelle les cellules sont traitées avec au moins un médicament principal connu pour être efficace dans le traitement du cancer.

3. Utilisation d'un composé selon la revendication 2, dans laquelle le médicament principal est la doxorubicine, le vinblastine, la vincristine, l'adriamycine, la mythramycine, l'actinomycine D, la colchicine, la daunomycine, la mitoxantrone ou la podopyllotoxine.

4. Utilisation d'un composé selon l'une quelconque des revendications 1 à 3, dans laquelle le niveau de dosage est d'environ 100 à 300 mg par jour.

5. Utilisation d'un composé selon l'une quelconque des revendications 1 à 4, dans laquelle le composé est la tétrandrine.
